# EUROPEAN PATENT SPECIFICATION

(11) **EP 0 506 205 B1**
(45) Date of publication and mention of the grant of the patent: **27.01.1999**
(21) Application number: 92201323.0
(22) Date of filing: 29.10.1985
(51) Int. Cl.: A61K 31/70

(54) **Use of adenosine for the manufacture of medicaments for continuous intravenous infusion to human patients**
Verwendung von Adenosin zur Herstellung eines Arzneimittels für kontinuierliche Intravenöse Infusion an menschlichen Patienten
Utilisation de l'adénosine pour la fabrication d'un médicament pour l'injection intraveineuse en continu chez l'homme

(30) Priority: 24.09.1985 US 779516
(43) Date of publication of application: 30.09.1992
(62) Divisional of application: 85905544.4
(73) Proprietor: ITEM DEVELOPMENT AB, S-182 71 Stocksund (SE)
(72) Inventor: Sollevi, Alf, S-161 55 Bromma (SE)
(74) Representative: Fogelberg, Lennart

(56) References cited:
- AMERICAN JOURNAL OF PHYSIOLOGY, vol. 219, no. 6, December 1970, pages 1672-1674; S. AFONSO et al.: "Inhibition of cardiovascular metabolic and hemodynamic effects of adenosine by aminophylline"
- CHEMICAL ABSTRACTS, vol. 97, 1982, page 573, abstract no. 89964r, Columbus, Ohio, US; S.E. DOWNING et al.: "Coronary dilator actions of adenosine and carbon dioxide in experimental diabetes", & AM. J. PHYSIOL. 1982, 243(2), H252-H258
- CARDIOVASCULAR RESEARCH, vol. 18, no. 4, April 1984, pages 220-228; H. GEWIRTZ et al.: "Influence of adenosine on basal myocardial function: observations in anaesthetised, domestic swine"
- JOURNAL OF NEUROSURGERY, vol. 58, no. 1, January 1983, pages 69-76; N.F. KASSELL et al.: "Cerebral ans systemic circulatory effects of arterial hypotension indeced by adenosine"
- CHEMICAL ABSTRACTS, vol. 57, 1982, page 467, abstract no. 142483m, Columbus, Ohio, US; M. NAKAMURA et al.: "Effect of adenosine and related vasoactive substances on blood flow in rat organs", & KYOTO-FURITSU IKA DAIGAKA ZASSHI 1982, 91(7), 523-35
- ANESTHESIOLOGY, vol. 61, October 1984, pages 400-405; A. SOLLEVI et al.: "Controlled hypotension with adenosine in cerebral aneurysm surgery"
- ANESTHESIOLOGY, vol. 60, June 1984, pages 547-552; M. LAGERKRANSER et al.: "Central and splanchic hemodynamics in the dog during controlled hypotension with adenosine"
- SCANDINAVIAN JOURNAL OF THORACIC AND CARDIOVASCULAR SURGERY, vol. 19, no. 2, 1985, pages 155-159; A. SOLLEVI et al.: "Adenosine spares platelets during cardiopulmonary bypass in man without causing systemic vasodilatation"
- CARDIOVASCULAR RESEARCH, vol. 13, no. 7, July 1979, pages 468-478; R.A. OLSSON et al.: "Coronary vasoactivity of adenosine in the conscious dog"

## Description

This invention is concerned with the use of adenosine for the manufacture of a medicament for the treatment of human beings. More particularly, this invention is concerned with the administration of adenosine to human patents by continuous intravenous infusion for providing preferential coronary vasodilation.

Adenosine is a naturally occurring nucleoside composed of the purine, adenine, and the sugar, D-ribose. Normal basal plasma levels of adenosine are from about 0.1 to about 0.2 µmol per liter. In addition, it is commonly present in the body in the form of adenosine monophosphate (AMP), adenosine diphosphate (ADP) and adenosine triphosphate (ATP). Adenosine has been reported to have a variety of biological effects, depending on whether the adenosine is endogenous or exogenously administered, including sedative and anti-epileptic effects on the central nervous system and inhibitory effects on respiration, cardio-vascular effects including prolongation of atrio-ventricular conduction time and impulse formation in the sinus node, vasodilation, antiaggregatory effect, decreased release of free fatty acids, anti-secretory effect in the stomach, and anti-diuretic effect.

As a general rule, however, adenosine and its biological effects have been largely of physiological interest. To the extent adenosine was of interest as a pharmaceutical product, that interest has centered primarily on its phosphate derivative, which now is known to be rapidly metabolized to yield adenosine and phosphate in the circulation. See Sollevi et al, Acta. Physiol. Scand. 120:171-6 (1984). However, phosphate may cause undesired side effects. For example, high levels of phosphate my cause arrhythmias secondary to chelation of magnesium and calcium. (See Dedrick, et al., Anesthesiology, 57:3A, 66 (1983).

Furthermore, adenosine is known to produce heart block through blockage of the atrioventricular (A-V) node. As a consequence, methylxanthines such as theophylline have been proposed by Berne, et al., in U.S. Patent No. 4 364 922 for use in preventing heart block caused by adenosine, in particular adenosine released as a consequence of cardiac ischemia or hypoxia.

In addition, it has been proposed to take advantage of adenosine's ability to block atrioventricular conductance by using it to treat tachyarrhythmias. For such use, adenosine is administered as an injectable intravenous bolus containing from about 37.5 micrograms/kg up to about 450 micrograms/kg of adenosine. In such a use, the adenosine has little detectable vasodilating action. Adenosine has a very short plasma half-life, of the order of 10-20 seconds (see, Fredholm and Sollevi,J. Physiol., 313:351-62 (1981)), and thus the concentration of injected adenosine is rapidly reduced to normal serum levels (about 0.15 µmol per liter). The transitory presence of the injected adenosine precludes all but the most transitory vasodilation.

Accordingly, for adenosine to be of practical value for use as a vasodilator, it must be administered continuously to maintain plasma levels sufficiently high to achieve vasodilation. The problem, however, is that such continuous administration could lead to undesired side effects, such as the above noted heart blockage.

It also should be noted that compounds commonly used as vasodilators, such as sodium nitroprusside, nitroglycerine, isoflurane, hydralazine, prazosin and the like, have various side effects. For example, sodium nitroprusside has the drawbacks of tachyphylaxis and rebound hypertension, apparently caused by autogenous generation of angiotensin to counteract the hypotensive effect of nitroprusside. As a consequence, the dosage of nitroprusside must be progressively increased with continued use to overcome the hypertensive effect of angiotensin, and there is a risk of rebound due to the presence of residual excess angiotensin. Nitroglycerine and prazosin suffer from the drawbacks of slow onset and unpredictable action. Isoflurane and sodium nitroprusside both have a tendency to reduce cardiac blood flow, while nitroprusside, hydralazine and prazosin increase heart rate.

Accordingly, there remains a need for a vasodilator suitable for administration by continous intravenous infusion.

Afonso et al (Am. J. of Physiol., Vol 219, No 6, December 1970, pp 1672-1674) reported a study of the vasodilator action of adenosine in dogs at the coronary and systemic level.

Downing et al (Chemical Abstracts, Vol 97, 1982, page 573, abstract No. 89964 r) studied the effect of adenosine on coronary sinus flow in lambs by means of dose-response curves focusing on actions only within the heart.

Gewirtz et al (Cardiovasc. Res., Vol 18, No 4, April 1984, pp 220-228) reported on intracoronary infusion of adenosine in swine in order to investigate a possible negative inotropic effect on the myocardium. Adenosine was administered in a high dose sufficient to cause maximal coronary vasodilatation.

Kassel et al (Journal of Neurosurgery, Vol 58, No 1, January 1983, pp 69-76) administered adenosine in dogs using very high doses after administration of a very high dose of dipyridamole which is a substance that potentiates the effect of adenosine, thus resulting in a lower dose of adenosine required in order to attain vascular effects.

Nakamura et al (Chemical Abstracts, Vol 57, 1982, page 467, abstract No 142483 m) investigated the effect of adenosine and related vasoactive substances on blood flow in rat organs.

Sollevi et al (Anesthesiology, Vol 61, October 1984, pp 400-405) studied the cardiovascular effect of adenosine-induced hypotension (reduced arterial blood pressure) in humans. Adenosine was administered by continuous infusion after dipyridamole having been infused. The measurements performed could not reveal regional vascular effects of adenosine infusion, nor is any such effect suggested.

Lagerkranser et al (Anesthesiology, Vol 60, June 1984, pp 547-552) studied central and splanchnic hemodynamic effects in dogs during controlled hypotension induced by the administration of adenosine and after previous administration of dipyridamole.

The present invention is based upon the discovery that adenosine can be administered to human patients under conditions such that an heretofore unappreciated activity profile is achieved, which differs significantly from the profiles of heretofore commonly used vasodilators. As a consequence of this discovery, it has been discovered that adenosine may be employed for the treatment of a variety of conditions by continuous intravenous infusion techniques. In particular, and as will be illustrated in greater detail below, adenosine has been found to have the following characteristics:
1. Adenosine has a preferential coronary vasodilatory effect in that it induces a considerably greater increase in blood flow within the myocardium than in other organs of the body when infused into the blood at doses which do not cause considerable reduction in blood pressure.
2. It improves coronary circulation during surgery in patients with ischemic heart disease.
3. It reduces the risk of coronary graft occlusion by increasing graft flow following coronary bypass surgery.
4. Adenosine is essentially non-toxic in the amounts used in accordance with the invention. It is readily taken up by the body to form ATP, and upon degradation its metabolites are present at or below levels normally resulting from physical exercise.

In accordance with this invention, adenosine may be administered to human patients by continuous intravenous infusion, without dipyridamole-pretreatment, to provide preferential coronary vasodilatation.

For purposes of this invention, adenosine can be administered to the patient in any pharmaceutically acceptable form suitable for use in continouos, intravenous infusion. A preferred form is an aqueous solution of adenosine, and more preferably adenosine in isotonic saline. The concentration of adenosine in the solution is not narrowly critical, although concentrations of at least 5 millimol (5 mM, or about 1,5 milligrams per milliliter, mg/ml) of solution are desired to avoid the need for excessive infusion rates to achieve desired serum levels. The concentration can be up to the solubility limit of adenosine (about 20 millimols per liter or 5.5 to 6 milligrams per milliliter) if desired.

Of course, the adenosine solution should be sterile and free from fungi and bacteria. Such solutions have been found to be stable at room temperature for at least two years.

Such solutions are prepared by mixing adenosine with the aqueous carrier, e.g. water or an isotonic solution, and other desired ingredients, to achieve a solution having the desired concentration, and thereafter sterilizing the solution.

Continous infusion can be achieved by any technique known to the art. Because adenosine has such a short plasma halflife and it is active at relatively low concentrations, it is desired that the method be one which minimizes or avoids fluctuations of serum adenosine levels.

The following examples illustrate in greater detail specific applications of continous intravenous infusion of adenosine in accordance with this invention.

### Example I

### Improved coronary circulation in ischemic heart disease

Patients requiring abdominal vascular surgery, such as surgery for an aortic aneurysm, frequently also suffer from ischemic heart disease, or insufficient blood flow to the heart tissue, which may present undesirable complications in such surgery. Accordingly, drugs with vasodilator properties, such as isoflurane and nitroprusside, have been investigated for possible use to increase myocardial blood flow and to reduce peripheral vascular resistance (after-load reduction) during such surgery; however, they have been found to have no beneficial effect with respect to coronary flow and, indeed, may reduce coronary blood flow. In contrast, adenosine administered by continuous infusion has been found very effective in increasing myocardial blood flow and, in such use, is accompanied by an increase in cardiac output.

For such application, the rate of adenosine administration should be such that there is no more than a 10-20 per cent reduction in blood pressure. As a general rule, this is achieved by use of rate of administration of the order of 0.05 to about 0.1 mg adenosine per kilogram per minute. In such a case myocardial blood flow has been found to be doubled, cardiac output has been increased by 10 to 20 per cent, and blood pressure has been reduced by 10 to 20 per cent, all without change in oxygen consumption and without ECG signs of ischemia.

### Example II

### Coronary vasodilation

It has been further found that when adenosine is administered by infusion at rates which do not induce significant hypotension, it has clinically useful regional effects in unanesthetized and anesthetized patients.

For example, adenosine at dosages of the order of 10 to 15 per cent of hypotensive levels (e.g. 0.02 to 0.05 mg per kg per minute) can be a useful adjunct to coronary by-pass surgery, apparently due to a preferential coronary vasodilation. It has been reported that coronary artery grafts occlude more frequently during the postoperative period when low graft flow values are obtained during surgery. See Groudin et al, Circulation, 42: Suppl 3: 106-111 (1970). It has been found that low doses of adenosine administered postoperatively increase graft blood flow without significant effect on atrioventricular conductance. The administration of low doses of adenosine for this purpose can be carried out for as long as is necessary to afford appropriate graft flows and reduce risk of occlusion, but ordinarily the period need not exceed 48 hours following surgery.

In a study designed to investigate the use of adenosine to inhibit occlusion of coronary grafts, nine patients (age 45-65, all taking beta-blockers) were studied during coronary artery surgery. After pre-medication, morphine (10-15 mg) and scopolamine (0.4-0.6 mg), anesthesia was induced by fentanyl (30 µg/kg b.w.). Pancuronium (0.1 mg/kg b.w.) was given to facilitate endotracheal intubation. Anesthesia was maintained with fentanyl 0.5 mg/hour, N₂O (50%) in oxygen and droperidol (0.1 mg/kg b.w.). During bypass thiomebumal (5 mg/kg b.w.) was given. Nitrous oxide was not used after bypass. Extracorporeal circulation (ECC) was performed with a roller pump and a Shiley buddle oxygenator primed with crystalloid solution. ECG (modified V₅), an arterial line and a Swan-Ganz Catheter were used for monitoring and for hemodynamic measurements. Blood flow in bypass grafts (n=15, internal mammary and venous grafts), was measured with appropriate sizes square wave electromagnetic flow probes (Nycotron 732). The study was performed 20-30 minutes after the termination of ECC. After a control Period (5 min), adenosine (5.3 mg/ml clinical solution) was continuously infused in a central vein in order to induce approximately 10 % reduction of mean arterial blood pressure (about 30 to 50 µg per kg per min). Graft flow was continuously measured before and during a 10 or 30 minute infusion of adenosine and finally during the following 5 minute control period. Data are expressed as mean ± SEN and differences were tested with Student's paired t-test against the preceding period.

The results of this study are summarized in Table I.

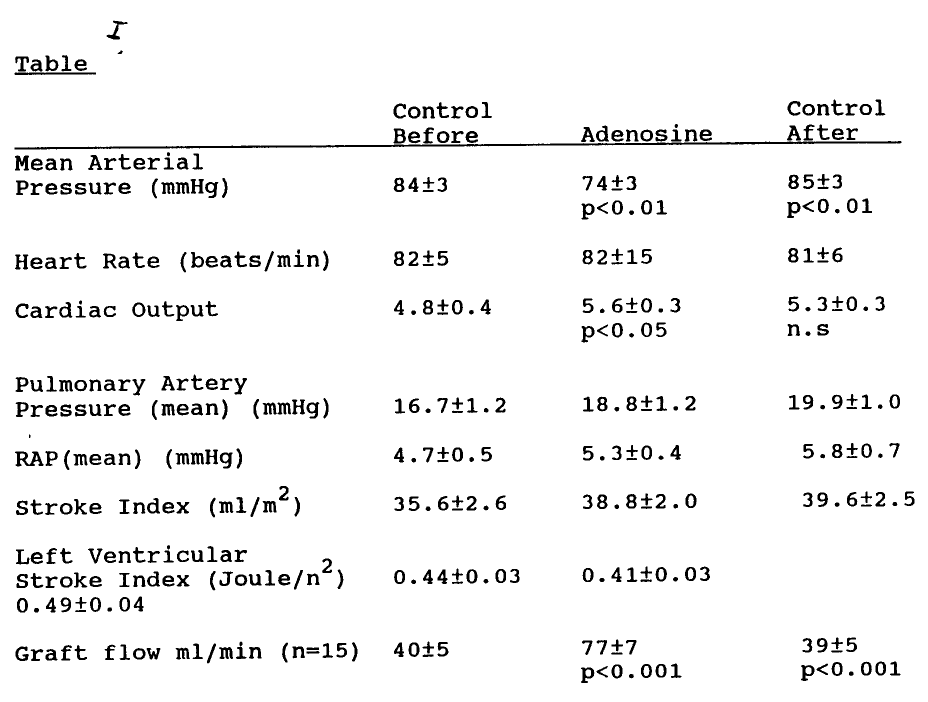

As is evident from Table I, adenosine in a dose of 40 ± 4 µg/kg/minute, a level which reduced mean arterial pressure 12 %, increased cardiac output 12 %, and doubled graft flow. At the same time, heart rate, mean pulmonary artery pressure, central venous pressure, stroke index and left ventricular stroke work index remained essentially unchanged. Graft flow rate was restored to its original value on termination of adenosine. No arrhythmias were observed.

This demonstrates that iv. adenosine at low rates (30-50 µg per kg per min) induces a marked and reproducible increase in graft flow without increased myocardial work, apparently due to preferential vasodilatory effect of adenosine in the coronary vasculature.

## Claims

1. Use of adenosine for the manufacture of a medicament for providing preferential coronary vasodilatation in humans by infusion at a rate low enough not to cause a reduction in blood pressure of more than 20 per cent.

2. Use as claimed in claim 1, wherein the medicament is manufactured for effecting increased blood flow in a coronary artery graft.

3. Use as claimed in claim 1 or claim 2, wherein the medicament is manufactured for infusion at a rate of 30 to 50 µg of adenosine per kilogram body weight per minute, when administered in a central vein.

4. Use as claimed in claim 1, wherein the medicament is manufactured for increasing myocardial blood flow in ischemic heart disease.

5. Use as claimed in claim 4, wherein the medicament is manufactured for infusion in a human patient at a rate of 50 to 100 µg of adenosine per kilogram body weight per minute, when administered in a central vein.

## Patentansprüche

1. Verwendung von Adenosin zur Herstellung eines Arzneimittels zur bevorzugten Koronargefäßerweiterung bei Menschen durch Infusion in einer genügend niedrigen Menge, um eine Verminderung des Blutdruckes von nicht mehr als 20 % hervorzurufen.

2. Verwendung nach Anspruch 1, bei der das Arzneimittel zur Bewirkung von erhöhtem Blutfluß in einem Koronararterientransplantat hergestellt wird.

3. Verwendung nach Anspruch 1 oder 2, bei der das Arzneimittel zur Infusion in einer Menge von 30 bis 50 µg/min Adenosin je 1 kg Körpergewicht bei Verabreichung in einer Zentralvene hergestellt wird.

4. Verwendung nach Anspruch 1, bei der das Arzneimittel zur Steigerung des Myokardblutflusses bei ischämischer Herzerkrankung hergestellt wird.

5. Verwendung nach Anspruch 4, bei der das Arzneimittel zur Infusion bei einem menschlichen Patienten in einer Menge von 50 bis 100 µg/min Adenosin je Kilogramm Körpergewicht bei Verabreichung in einer Zentralvene hergestellt wird.

## Revendications

1. Utilisation d'adénosine pour la fabrication d'un médicament destiné à procurer une vasodilatation coronarienne préférentielle chez les humains, par perfusion à une vitesse suffisamment faible pour ne pas provoquer une réduction de plus de 20 pour cent de la pression sanguine.

2. Utilisation selon la revendication 1, dans laquelle le médicament est fabriqué pour augmenter le débit sanguin dans une greffe d'artère coronaire.

3. Utilisation selon la revendication 1 ou la revendication 2, dans laquelle le médicament est fabriqué pour être perfusé à une vitesse de 30 à 50 µg d'adénosine par kilogramme de poids corporel et par minute, lorsqu'il est administré dans une veine centrale.

4. Utilisation selon la revendication 1, dans laquelle le médicament est fabriqué pour augmenter le débit sanguin du myocarde dans le cas d'une cardiopathie ischémique.

5. Utilisation selon la revendication 4, dans laquelle le médicament est fabriqué pour être perfusé chez un patient humain à une vitesse de 50 à 100 µg d'adénosine par kilogramme de poids corporel et par minute, lorsqu'il est administré dans une veine centrale.
